(19) Europäisches Patentamt
European Patent Office
Office européen des brevets

(11) EP 1 919 850 B1

(12) **EUROPEAN PATENT SPECIFICATION**

(45) Date of publication and mention of the grant of the patent:
**18.03.2009 Bulletin 2009/12**

(21) Application number: **06789700.9**

(22) Date of filing: **11.08.2006**

(51) Int Cl.:
*C07C 2/32* (2006.01) *C07D 213/53* (2006.01)
*C07F 15/02* (2006.01)

(86) International application number:
**PCT/US2006/031410**

(87) International publication number:
**WO 2007/021955 (22.02.2007 Gazette 2007/08)**

(54) **NITRO SUBSTITUTED ALPHA-OLEFIN SYNTHESIS CATALYSTS**

KATALYSATOREN FÜR DIE SYNTHESE NITROSUBSTITUIERTER ALPHA-OLEFINE

CATALYSEURS POUR LA SYNTHÈSE D'ALPHA-OLEFINES NITRO SUBSTITUÉES

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.08.2005 US 707632 P**

(43) Date of publication of application:
**14.05.2008 Bulletin 2008/20**

(73) Proprietor: **E.I. DU PONT DE NEMOURS AND COMPANY**
**Wilmington, DE 19898 (US)**

(72) Inventors:
- **ADELMAN, Douglas, J.**
  **Wilmington, Delaware 19810 (US)**
- **IONKIN, Alex, Seregy**
  **Kennett Square, Pennsylvania 19348 (US)**

(74) Representative: **Towler, Philip Dean**
**Frank B. Dehn & Co.**
**St Bride's House**
**10 Salisbury Square**
**London**
**EC4Y 8JD (GB)**

(56) References cited:
**WO-A-01/58874**

- **IONKIN ET AL: "nitro-substituted iron (II) tridentate bis(imino)pyridine complexes as high temperature catalysts for the production of alpha olefins" JOURNAL OF POLYMER SCIENCE, POLYMER CHEMISTRY EDITION., vol. 44, no. 8, 2006, pages 2615-2635, XP002417531 USJOHN WILEY AND SONS. NEW YORK.**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 919 850 B1

## Description

### FIELD OF THE INVENTION

Iron, cobalt, chromium or vanadium complexes of certain 2,6-pyridinecarboxaldehydes and 2,6-diacylpyridines in which nitro groups are substituted on an aryl group bound to an imino nitrogen atom have longer catalytic lives when used as catalysts to produce α-olefins by oligomerization of ethylene.

### TECHNICAL BACKGROUND

α-Olefins are important items of commerce, hundreds of millions of kilograms being manufactured yearly. They are useful as monomers for (co)polymerizations and as chemical intermediates for the manufacture of many other materials, for example detergents and surfactants. α-Olefins are most commonly made by the oligomerization of ethylene. Many types of catalysts for this reaction are known, among them certain transition metal complexes of diimines of 2,6-pyridinecarboxaldehydes and 2,6-diacylpyridines and related compounds, see for instance US6103946, US6534691, US6555723, US6683187 and US 6710006, and WO04/026795.

As with any catalyst system one is normally concerned about the length of time the catalyst remains active and/or retains a good percentage of its activity over a prolonged period. The diimine complex catalysts mentioned above often have good activity but this diminishes as the temperatures of the process is raised, particularly above about 80-100°C. Therefore such catalysts with improved and/or prolonged activities, especially at higher temperatures, are desired.

### SUMMARY OF THE INVENTION

This invention concerns a process for the oligomerization of ethylene to linear α-olefins (LAOs), comprising contacting, at a temperature of -20°C to 200°C, ethylene and a Fe, Co, Cr or V complex of a ligand of the formula

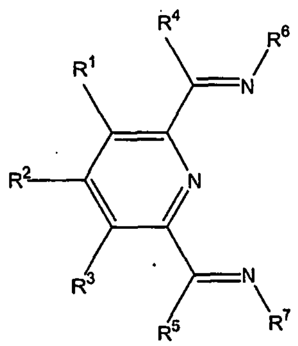
(I)

wherein:

$R^1$, $R^2$ and $R^3$ are each independently hydrogen, hydrocarbyl, substituted hydrocarbyl or an inert functional group, provided that any two of $R^1$, $R^2$ and $R^3$ vicinal to one another taken together may form a ring;
$R^4$ and $R^5$ are each independently hydrogen, hydrocarbyl, substituted hydrocarbyl or an inert functional group provided that $R^1$ and $R^4$ and/or $R^3$ and $R^5$ taken together may form a ring;
$R^6$ and $R^7$ are each independently aryl or substituted aryl having a first ring atom bound to the imino nitrogen, provided that at least one of $R^6$ and $R^7$ is substituted with at least one nitro group.

Also disclosed herein is a compound of the formula

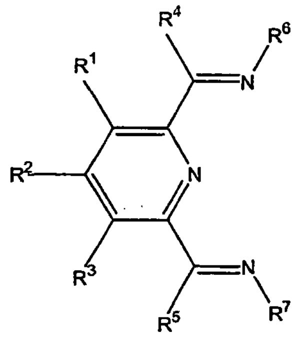

wherein:

$R^1$, $R^2$ and $R^3$ are each independently hydrogen, hydrocarbyl, substituted hydrocarbyl or an inert functional group, provided that any two of $R^1$, $R^2$ and $R^3$ vicinal to one another taken together may form a ring;
$R^4$ and $R^5$ are each independently hydrogen, hydrocarbyl, substituted hydrocarbyl or an inert functional group provided that $R^1$ and $R^4$ and/or $R^3$ and $R^5$ taken together may form a ring;
$R^6$ and $R^7$ are each independently aryl or substituted aryl having a first ring atom bound to the imino nitrogen, provided that at least one of $R^6$ and $R^7$ is substituted with at least one nitro group, and provided that:

in $R^6$, a second ring atom adjacent to said first ring atom is bound to a halogen, a primary carbon group, a secondary carbon group or a tertiary carbon group; and further provided that
in $R^6$, when said second ring atom is bound to a halogen or a primary carbon group, none, one or two of the other ring atoms in $R^6$ and $R^7$ adjacent to said first ring atom are bound to a halogen or a primary carbon group, with the remainder of the ring atoms adjacent to said first ring atom being bound to a hydrogen atom; or
in $R^6$, when said second ring atom is bound to a secondary carbon group, none, one or two of the other ring atoms in $R^6$ and $R^7$ adjacent to said first ring atom are bound to a halogen, a primary carbon group or a secondary carbon group, with the remainder of the ring atoms adjacent to said first ring atom being bound to a hydrogen atom; or
in $R^6$, when said second ring atom is bound to a tertiary carbon group, none or one of the other ring atoms in $R^6$ and $R^7$ adjacent to said first ring atom are bound to a tertiary carbon group, with the remainder of the ring atoms adjacent to said first ring atom being bound to a hydrogen atom.

This invention also concerns a compound of the formula

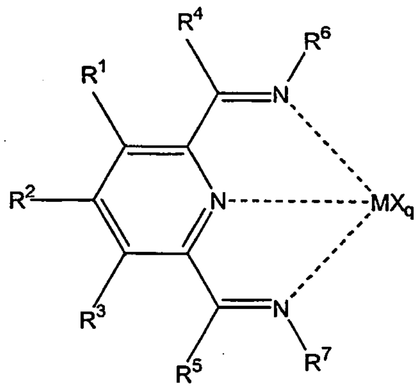

wherein:

each X is independently a monoanion;
M is a transition metal selected from the group consisting of Fe, Co, Cr and V;
q is an oxidation state of said transition metal;
$R^1$, $R^2$ and $R^3$ are each independently hydrogen, hydrocarbyl, substituted hydrocarbyl or an inert functional group,

provided that any two of $R^1$, $R^2$ and $R^3$ vicinal to one another taken together may form a ring;
$R^4$ and $R^5$ are each independently hydrogen, hydrocarbyl, substituted hydrocarbyl or an inert functional group provided that $R^1$ and $R^4$ and/or $R^3$ and $R^5$ taken together may form a ring;
$R^6$ and $R^7$ are each independently aryl or substituted aryl having a first ring atom bound to the imino nitrogen, provided that at least one of $R^6$ and $R^7$ is substituted with at least one nitro group, and provided that: in $R^6$, a second ring atom adjacent to said first ring atom is bound to a halogen, a primary carbon group, a secondary carbon group or a tertiary carbon group; and further provided that
in $R^6$, when said second ring atom is bound to a halogen or a primary carbon group, none, one or two of the other ring atoms in $R^6$ and $R^7$ adjacent to said first ring atom are bound to a halogen or a primary carbon group, with the remainder of the ring atoms adjacent to said first ring atom being bound to a hydrogen atom; or
in $R^6$, when said second ring atom is bound to a secondary carbon group, none, one or two of the other ring atoms in $R^6$ and $R^7$ adjacent to said first ring atom are bound to a halogen, a primary carbon group or a secondary carbon group, with the remainder of the ring atoms adjacent to said first ring atom being bound to a hydrogen atom; or
in $R^6$, when said second ring atom is bound to a tertiary carbon group, none or one of the other ring atoms in $R^6$ and $R^7$ adjacent to said first ring atom are bound to a tertiary carbon group, with the remainder of the ring atoms adjacent to said first ring atom being bound to a hydrogen atom.

This invention also includes the ligands (I), and their complexes.

## DETAILS OF THE INVENTION

Herein certain terms are used, and many of them are defined below.
A "hydrocarbyl group" is a univalent group containing only carbon and hydrogen. As examples of hydrocarbyls may be mentioned unsubstituted alkyls, cycloalkyls and aryls. If not otherwise stated, it is preferred that hydrocarbyl groups (and alkyl groups) herein contain 1 to about 30 carbon atoms.
By "substituted hydrocarbyl" herein is meant a hydrocarbyl group that contains one or more substituent groups that are inert under the process conditions to which the compound containing these groups is subjected (e.g., an inert functional group, see below). The substituent groups also do not substantially detrimentally interfere with the polymerization process or operation of the polymerization catalyst system. If not otherwise stated, it is preferred that (substituted) hydrocarbyl groups herein contain 1 to about 30 carbon atoms. Included in the meaning of "substituted" are rings containing one or more heteroatoms, such as nitrogen, oxygen and/or sulfur, and the free valence of the substituted hydrocarbyl may be to the heteroatom. In a substituted hydrocarbyl, all of the hydrogens may be substituted, as in trifluoromethyl.
By "(inert) functional group" herein is meant a group, other than hydrocarbyl or substituted hydrocarbyl, which is inert under the process conditions to which the compound containing the group is subjected. The functional groups also do not substantially deleteriously interfere with any process described herein that the compound in which they are present may take part in. Examples of functional groups include halo (fluoro, chloro, bromo and iodo), and ether such as $-OR^{50}$ wherein $R^{50}$ is hydrocarbyl or substituted hydrocarbyl. In cases in which the functional group may be near a transition metal atom, the functional group alone should not coordinate to the metal atom more strongly than the groups in those compounds that are shown as coordinating to the metal atom, which is they should not displace the desired coordinating group.
By a "cocatalyst" or a "catalyst activator" is meant one or more compounds that react with a transition metal compound to form an activated catalyst species. One such catalyst activator is an "alkylaluminum compound" which, herein, means a compound in which at least one alkyl group is bound to an aluminum atom. Other groups such as, for example, alkoxide, hydride, an oxygen atom bridging two aluminum atoms and halogen may also be bound to aluminum atoms in the compound.
By a "linear $\alpha$-olefin product" is meant a composition predominantly comprising a compound or mixture of compounds of the formula $H(CH_2CH_2)_qCH=CH_2$ wherein q is an integer of 1 to about 18. In most cases, the linear $\alpha$-olefin product of the present process will be a mixture of compounds having differing values of q of from 1 to 18, with a minor amount of compounds having q values of more than 18. Preferably less than 50 weight percent, and more preferably less than 20 weight percent, of the product will have q values over 18. The product may further contain small amounts (preferably less than 30 weight percent, more preferably less than 10 weight percent, and especially preferably less than 2 weight percent) of other types of compounds such as alkanes, branched alkenes, dienes and/or internal olefins.
By a "primary carbon group" herein is meant a group of the formula $-CH_2$---, wherein the free valence --- is to any other atom, and the bond represented by the solid line is to a ring atom of a substituted aryl to which the primary carbon group is attached. Thus the free valence --- may be bonded to a hydrogen atom, a halogen atom, a carbon atom, an oxygen atom, a sulfur atom, etc. In other words, the free valence --- may be to hydrogen, hydrocarbyl, substituted hydrocarbyl or a functional group. Examples of primary carbon groups include $-CH_3$, $-CH_2CH(CH_3)_2$, $-CH_2Cl$, $-CH_2C_6H_5$,

$-OCH_3$ and $-CH_2OCH_3$.

By a "secondary carbon group" is meant the group

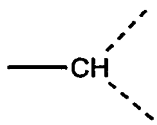

wherein the bond represented by the solid line is to a ring atom of a substituted aryl to which the secondary carbon group is attached, and both free bonds represented by the dashed lines are to an atom or atoms other than hydrogen and fluorine. These atoms or groups may be the same or different. In other words the free valences represented by the dashed lines may be hydrocarbyl, substituted hydrocarbyl or inert functional groups. Examples of secondary carbon groups include $-CH(CH_3)_2$, $-CHCl_2$, $-CH(C_6H_5)_2$, cyclohexyl, $-CH(CH_3)OCH_3$, and $-CH=CHCH_3$.

By a "tertiary carbon group" is meant a group of the formula

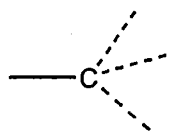

wherein the bond represented by the solid line is to a ring atom of a substituted aryl to which the tertiary carbon group is attached, and the three free bonds represented by the dashed lines are to an atom or atoms other than hydrogen and fluorine. In other words, the bonds represented by the dashed lines are to hydrocarbyl, substituted hydrocarbyl or inert functional groups. Examples of tetiary carbon groups include $-C(CH_3)_3$, $-C(C_6H_5)_3$, $-CCl_3$, $-C(CH_3)_2OCH_3$, $-C\equiv CH$, $-C(CH_3)_2CH=CH_2$, aryl and substituted aryl such as phenyl, and 1-adamantyl.

By relatively noncoordinating (or weakly coordinating) anions are meant those anions as are generally referred to in the art in this manner, and the coordinating ability of such anions is known and has been discussed in the literature, see for instance W. Beck., et al., Chem. Rev., vol. 88 p. 1405-1421 (1988), and S. H. Stares, Chem. Rev., vol. 93, p. 927-942 (1993), both of which are hereby included by reference. Among such anions are those formed from the aluminum compounds in the immediately preceding paragraph and $X^-$ including $R^9_3AlX^-$, $R^9_2AlClX^-$, $R^9AlCl_2X^-$, and "$R^9AlOX^-$", wherein $R^9$ is alkyl. Other useful noncoordinating anions include $BAF^-$ {BAF = tetrakis[3,5-bis(trifluoromethyl)phenyl] borate}, $SbF_6^-$, $PF_6^-$, and $BF_4^-$, trifluoromethanesulfonate

By a "monoanionic ligand" is meant a ligand with one negative charge.

By a "neutral ligand" is meant a ligand that is not charged.

"Alkyl group" and "substituted alkyl group" have their usual meaning (see above for substituted under substituted hydrocarbyl). Unless otherwise stated, alkyl groups and substituted alkyl groups preferably have 1 to about 30 carbon atoms.

By "aryl" is meant a monovalent aromatic group in which the free valence is to the carbon atom of an aromatic ring. An aryl may have one or more aromatic rings, which may be fused, connected by single bonds or other groups.

By "substituted aryl" is meant a monovalent aromatic group substituted as set forth in the above definition of "substituted hydrocarbyl". Similar to an aryl, a substituted aryl may have one or more aromatic rings, which may be fused, connected by single bonds or other groups; however, when the substituted aryl has a heteroaromatic ring, the free valence in the substituted aryl group can be to a heteroatom (such as nitrogen) of the heteroaromatic ring instead of a carbon.

By "oligomerization conditions" herein is meant conditions for causing olefin oligomerization with catalysts described herein. Such conditions may include temperature, pressure, oligomerization method such as liquid phase, continuous, batch, and the like. Also included may be cocatalysts which are needed and/or desirable.

In (I) and its Fe, Co, Cr or V complexes the structures of $R^6$ and $R^7$ are particularly important in determining the Schulz-Flory constant of the mixtures of LAOs produced. This is a measure of the molecular weights of the olefins obtained, usually denoted as factor K, from the Schulz-Flory theory (see for instance B. Elvers, et al., Ed. Ullmann's Encyclopedia of Industrial Chemistry, Vol. A13, VCH Verlagsgesellschaft mbH, Weinheim, 1989, p. 243-247 and 275-276). This is defined as:

$$K = n(C_{n+2}\text{ olefin})/n(C_n\text{ olefin})$$

wherein n($C_n$ olefin) is the number of moles of olefin containing n carbon atoms, and n($C_{n+2}$ olefin) is the number of moles of olefin containing n+2 carbon atoms, or in other words the next higher oligomer of $C_n$ olefin. From this can be determined the weight (mass) fractions of the various olefins in the resulting oligomeric reaction product mixture. The K factor is usually preferred to be in the range of about 0.6 to about 0.8 to make the $\alpha$-olefins of the most commercial interest. It is also important to be able to vary this factor, so as to produce those olefins, which are in demand at the moment.

Preferably $R^6$ and $R^7$ are each independently aryl or substituted aryl. In $R^6$ and $R^7$ it is further preferred that:

in $R^6$, a second ring atom adjacent to said first ring atom is bound to a halogen, a primary carbon group, a secondary carbon group or a tertiary carbon group; and further provided that

in $R^6$, when said second ring atom is bound to a halogen or a primary carbon group, none, one or two of the other ring atoms in $R^6$ and $R^7$ adjacent to said first ring atom are bound to a halogen or a primary carbon group, with the remainder of the ring atoms adjacent to said first ring atom being bound to a hydrogen atom; or

in $R^6$, when said second ring atom is bound to a secondary carbon group, none, one or two of the other ring atoms in $R^6$ and $R^7$ adjacent to said first ring atom are bound to a halogen, a primary carbon group or a secondary carbon group, with the remainder of the ring atoms adjacent to said first ring atom being bound to a hydrogen atom; or

in $R^6$, when said second ring atom is bound to a tertiary carbon group, none or one of the other ring atoms in $R^6$ and $R^7$ adjacent to said first ring atom are bound to a tertiary carbon group, with the remainder of the ring atoms adjacent to said first ring atom being bound to a hydrogen atom.

In one preferred form $R^6$ is

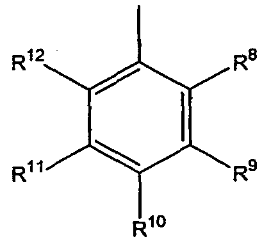

(II)

and $R^7$ is

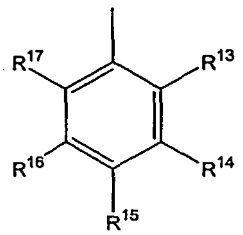

(III)

wherein these groups are substituted as described above. Any of the positions in (II) and (III) may be substituted with nitro, and when substituted adjacent to the first ring atom (i.e., in an ortho position) it is considered a secondary carbon group for the purposes above. However it is preferred that the nitro group(s) be substituted in the $R^9$, $R^{10}$, $R^{11}$, $R^{14}$, $R^{15}$, and/or $R^{16}$ positions, and especially preferably in or both of the $R^{10}$ and $R^{15}$ positions (para to the first ring atom). It is also preferred that two or more nitro groups be present. In a specific preferred form at least one nitro group is present in each of $R^6$ and $R^7$, more preferably one nitro group is present in each of $R^6$ and $R^7$, and even more preferably in $R^6$ it is present in one of the $R^9$, $R^{10}$ or $R^{11}$ positions, and in $R^7$ it is present in one of the $R^{14}$, $R^{15}$ or $R^{16}$ positions. Another preferred position for a nitro group is $R^2$.

In (II) and (III) $R^9$, $R^{10}$, $R^{11}$, $R^{14}$, $R^{15}$ and $R^{16}$ are each independently hydrogen, hydrocarbyl, substituted hydrocarbyl or an inert functional group (including nitro as described above), and provided that any two of $R^8$, $R^9$, $R^{10}$, $R^{11}$, $R^{12}$, $R^{13}$, $R^{14}$, $R^{15}$, $R^{16}$ and $R^{17}$ that are vicinal to one another, taken together may form a ring.

(II) and (III) may be identical (so that substitution on the imino nitrogen atoms is "symmetric") or they may be different, including different in the ortho positions (so that the substitution on the imino nitrogen atoms is "asymmetric").

In another preferred variant of (I), $R^6$ and $R^7$ are each independently a substituted 1-pyrrolyl. More preferably

in this instance, $R^6$ and $R^7$ are, respectively

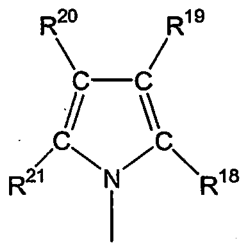 (IV) and 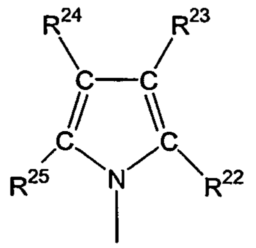 (V),

wherein:

$R^{18}$ and $R^{21}$ correspond to the definitions of, and preferences for, $R^8$ and $R^{12}$ in (II);
$R^{22}$ and $R^{25}$ correspond to the definitions of, and preferences for, $R^{13}$ and $R^{17}$ in (III);
$R^{19}$ and $R^{20}$ correspond to the definitions of, and preferences for, $R^9$ and $R^{11}$ in (II); and
$R^{23}$ and $R^{24}$ correspond to the definitions of, and preferences for, $R^{14}$ and $R^{16}$ in (III).

In (IV) and (V) it is preferred that the nitro group(s) is in the $R^{19}$, $R^{20}$, $R^{23}$ and $R^{24}$ positions.

By a "first ring atom in $R^5$ and $R^6$ bound to an imino nitrogen atom" is meant the ring atom in these groups bound to an imino nitrogen shown in (I), for example

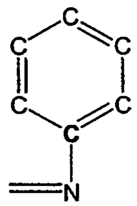 (VI) or 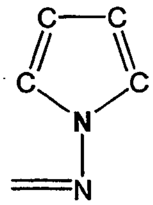 (VII)

the atoms shown in the 1-position in the rings in (VI) and (VII) are the first ring atoms bound to an imino carbon atom (other groups which may be substituted on the aryl groups are not shown). Ring atoms adjacent to the first ring atoms are shown, for example, in (VIII) and (IX), where the open valencies to these adjacent atoms are shown by dashed lines [the 2,6-positions in (VIII) and the 2,5-positions in (IX)].

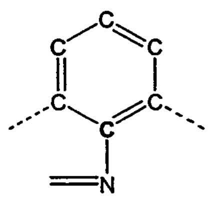 (VIII) or 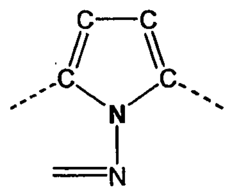 (IX)

In groups (II) and (III), it is particularly preferred that:

if $R^8$ is a primary carbon group or halogen, $R^{13}$ is a primary carbon group or halogen, and $R^{12}$ and $R^{17}$ are hydrogen; or
if $R^8$ is a secondary carbon group, $R^{13}$ is a primary carbon group, halogen or a secondary carbon group, more preferably a secondary carbon group, and $R^{12}$ and $R^{17}$ are hydrogen; or
if $R^8$ is a tertiary carbon group (more preferably a trihalo tertiary carbon group such as a trihalomethyl), and $R^{12}$, $R^{13}$ and $R^{17}$ are hydrogen.

In the present complexes and their use as oligomerization catalysts, a preferred transition metal is iron. Preferably the oxidation state of the iron atom is +2.

Generally speaking the present complexes are effective oligomerization catalysts from about -20°C to about

200°C, preferably about 0°C to about 150°C, and more preferably about 80°C to about 150°C. The pressure (if one or more monomers such as ethylene are gaseous) is not critical, atmospheric pressure to 70 MPa being a useful range. Any combination of temperature and pressure may be used. The particular combination of temperature and pressure chosen will reflect many factors, including oligomer yield, type of oligomerization process being used, the relative economics of various conditions, etc. A cocatalyst is often added which is an alkylating are hydriding agent. A preferred type of cocatalyst is an alkylaluminum compound, and preferred alkylaluminum compounds are methylaluminoxane, trimethylaluminum, and other trialkylaluminum compounds. Especially preferred alkylaluminum compounds are methylaluminoxane, trimethylaluminum.

Useful forms of complexes of (II) include those of the following formulas (written as iron complexes, but analogous structures may be written for Co, Cr and V):

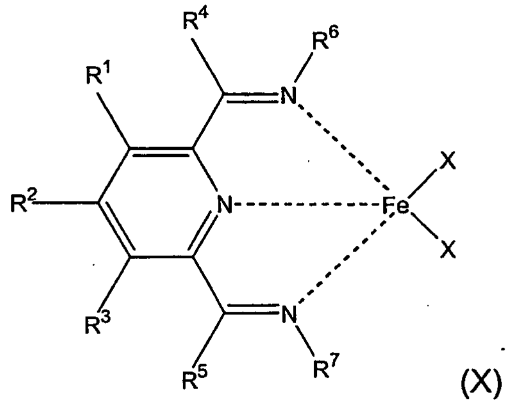

(X)

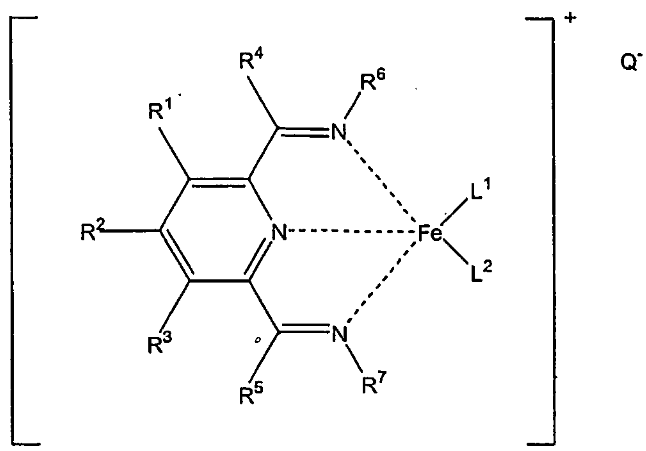

(XI),

and

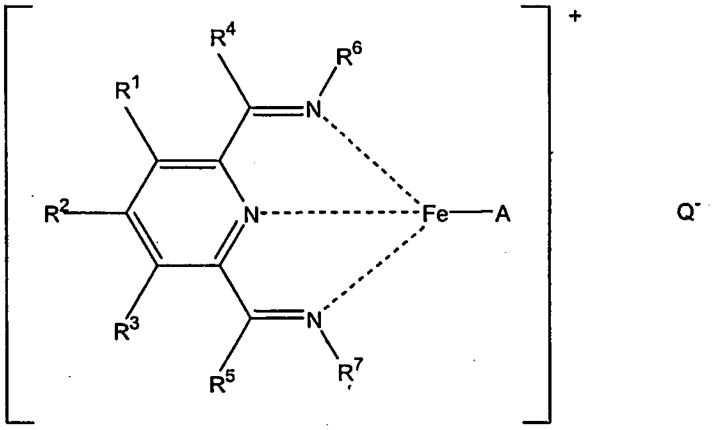

(XII),

wherein:

$R^1$, $R^2$, $R^3$, $R^4$, $R^5$, $R^6$, and $R^7$ are as defined above;
each X is independently a monoanion;
A is a π-allyl or π-benzyl group;
$L^1$ is a neutral monodentate ligand which may be displaced by said olefin or an open coordination site, and $L^2$ is a monoanionic monodentate ligand which preferably can add to an olefin, or $L^1$ and $L^2$ taken together are a monoanionic bidentate ligand, provided that said monoanionic monodentate ligand or said monoanionic bidentate ligand may add to said olefin; and
Q is a relatively noncoordinating anion.

Preferably each X is independently halide or carboxylate, more preferably both of X are chloride or bromide.

When A is present, in effect $L^1$ and $L^2$ taken together are A. (X) may be used as a starting material for forming an active polymerization complex. For example (X) may be reacted with:

(a) a first compound W, which is a neutral Lewis acid capable of abstracting $X^-$ and alkyl group or a hydride group from M to form $WX^-$, $(WR^{40})^-$ or $WH^-$, wherein $R^{40}$ is alkyl, and which is also capable of transferring an alkyl group or a hydride to M, provided that $WX^-$ is a weakly coordinating anion; or
(b) a combination of second compound which is capable of transferring an alkyl or hydride group to M and a third compound which is a neutral Lewis acid which is capable of abstracting $X^-$, a hydride or an alkyl group from M to form a weakly coordinating anion.

Thus W may be an alkylaluminum compound, while the second compound may be a dialkylzinc compound and the third compound may be a borane such as tris(pentafluorophenyl)borane. Such combinations and compounds for W are well known in the art, see for instance U.S. Patents 5,955,555 and 5,866,663, both of which are hereby included by reference.

In many instances compounds (XI) or (XII), if added directly to an oligomerization process, may be active catalysts without the addition of any cocatalysts, or just the addition of a Lewis acid which may form a relatively noncoordinating anion by abstraction of $A^-$ or $L^{2-}$ from the complex. In (XI) $L^2$ may be an alkyl group, which is in fact an oligomer or polymer of the olefinic monomer being polymerized, while $L^1$ may be an open coordination site or $L^1$ is one of the olefins being polymerized. For example if ethylene is being oligomerized, $L^2$ may be $-(CH_2CH_2)_zD$ wherein z is a positive integer and D is an anion (which originally was $L^2$) between which ethylene could insert between $L^2$ and the Fe atom. In effect under these circumstances (XII) could be thought of as a so-called living polymer. The chemistry of such types of compounds is known, see for instance U.S. Patents 5,866,663 and 5,955,555, both of which are hereby included by reference.

The oligomerization process may be a batch, semibatch or continuous process, and a continuous process is preferred. Typically the process is carried out as a solution process, either with a separate solvent or using the LAOs as produced as the solvent. These types of processes are well known in the art. For example a solution process may be carried out in one or more continuous stirred tank reactors (CSTR), or a pipeline reactor. Such processes and conditions for the oligomerization are described in US6103946, US6534691, US6555723, US6683187 and US 6710006, and WO04/026795.

Useful analogous polymerization catalysts in which the aryl groups attached to the imino carbon atoms are

substituted with nitro groups in the same patterns as described above may also be made and used to form polyolefins, especially polyethylene. Such catalysts (less the nitro groups) are described in US5955555. Similar nitro substituted pyrrolyl groups may also be present in catalysts for polymerizations.

In the Examples all air-sensitive compounds were prepared and handled under a $N_2$/Ar atmosphere using standard Schlenk and inert-atmosphere box techniques. Anhydrous solvents were used in the reactions. Solvents were distilled from drying agents or passed through columns under an argon or nitrogen atmosphere. Anhydrous iron(II) chloride, 1-(6-acetyl-pyridin-2-yl)-ethanone, 2-methyl-4-nitro-phenylamine, 4-nitro-phenylamine, 2,4,6-trimethylphenylamine, 2-methyl-3-nitro-phenylamine, 2-methyl-4-diethylaminophenylamine monochloride, potassium acetate, n-butanol were purchased from Sigma-Aldrich (St. Louis, MO 63103, USA). 2,6-Bis(1-(2-methylphenylimino)ethyl)pyridine was prepared according to WO98/27124. In the Examples THF is tetrahydrofuran.

Experiment 1

2,6-Bis(1-(2-methylphenylimino)ethyl)pyridine iron (II) chloride (1)

Anhydrous iron(II) chloride (1.6 g, 0.0126 mol) was dissolved in 100 ml warm *n*-butanol. Then, 4.53 g (0.0133 mol) of 2,6-bis(1-(2-methylphenylimino)ethyl)pyridine was added in one portion in the reaction mixture. The mixture was kept at 40°C for an additional h and was cooled to ambient temperature. The resultant blue precipitate was filtered and washed twice with 40 ml of pentane and dried under vacuum. The yield of 1 was 4.1 g (69%). Anal. Calculated for $C_{23}H_{23}Cl_2FeN_3$ (Mol. Wt.: 468.20): C, 59.00; H, 4.95; N, 8.97. Found: C, 59.24; H, 5.07; N, 9.14. Direct probe MS: Exact Mass for $C_{23}H_{23}Cl_2FeN_3$: 467.06. Found 467.06.

Example 1

2,6-Bis(1-(2-methyl-4-nitrophenylimino)ethyl)pyridine (6)

1-(6-Acetyl-pyridin-2-yl)-ethanone (10.0 g, 0.0613 mol), 19.58 g (0.129 mol) of 2-methyl-4-nitro-phenylamine, 300 ml of the toluene and a few crystals of *p*-toluenesulfonic acid were refluxed under the flow of the nitrogen while using a Dean-Stark trap for 3 days until the calculated amount of the water was separated (2.21 ml). The solvent was removed in a rotary evaporator and the resultant reaction mixture was recrystallized from 50 ml of ethanol. The yield of **6** was 18.71 g (71 %) as a pale yellow solid. Compound **6** is stable at the room temperature. $^1H$ NMR (500 MHz, $CD_2Cl_2$, TMS): $\delta$ 1.95 (s, 6 H, Me), 2.06 (s, 6 H, Me), 6.70 (s, 2H, Arom-H), 7.48 (m, 4H, Arom-H), 8.12 (s, 2H, Py-H), 8.20 (s, 1H, Py-H). $^{13}C$ NMR (500 MHz, $CD_2Cl_2$ (selected signals)): $\delta$ 167.9 (C=N). Anal. Calculated for $C_{23}H_{21}N_5O_4$ (Mol. Wt.: 431.44): C, 64.03; H, 4.91; N. 16.23. Found: C, 64.28; H, 5.14; N. 16.20.

Example 2

1-{6-[1-(2,4,6-Trimethyl-phenlimino)-ethyl]-pyridin-2-yl}-ethanone (**13**)

1-(6-Acetyl-pyridin-2-yl)-ethanone (20.0 g, 0.123 mol), 14.92 g (0.110) of 2,4,6-trimethylphenylamine and 300 ml of *n*-propanol with a few crystals of *p*-toluenesulfonic acid were stirred at room temperature for 36 hours in 500 ml flask under the flow of the nitrogen. The resultant yellow precipitate was filtered and washed with 20 ml of methanol. It was then dried under vacuum overnight. The yield of **13** was 9.01 g (26%) as a yellow solid. $^1H$ NMR (500 MHz, $CD_2Cl_2$, TMS): $\delta$ 1.90 (s, 6 H, Me), 2.20 (s, 3 H, Me), 2.28 (s, 3 H, Me), 2.75 (s, 3 H, Me), 6.90 (s, 2H, Arom-H), 7.90 (t, $^3J_{HH}$=8.0 Hz, 1H, Py-H), 8.09 (d, $^3J_{HH}$=8.0 Hz, 1H, Py-H), 8.55 (d, $^3J_{HH}$=8.0 Hz, 1H, Py-H). $^{13}C$ NMR (500 MHz, $CD_2Cl_2$, TMS (selected signals)): $\delta$ 167.3 (C=N), 200.0 (C=O). Anal. Calculated for $C_{18}H_{20}N_2O$ (Mol. Wt.: 280.36): C, 77.11; H, 7.19; N, 9.99. Found: C, 77.13; H, 7.20; N, 10.20.

Example 3

(1-{6-[1-(4-Nitro-phenylimino)-ethyl]-pyridin-2-yl}-ethylidene)-(2,4,6-trimethyl-phenyl)-amine (**8**)

1-{6-[1-(2,4,6-Trimethyl-phenylimino)-ethyl]-pyridin-2-yl}-ethanone 13 (5.0 g, 0.0178 mol), 2.58 g (0.0187 mol) of 4-nitro-phenylamine, 100 g of fresh molecular sieves and 100 ml of toluene were kept at 90°C for 3 days under a flow of nitrogen. The solvent was removed in a rotary evaporator and the residue was recrystallized from 10 ml of ethanol. The yield of **8** was 3.80 g (53%) as a yellow solid. $^1H$ NMR (500 MHz, $CD_2Cl_2$, TMS): $\delta$ 2.05 (s, 6 H, Me), 2.20 (s, 3 H, Me), 2.30 (s, 3 H, Me), 2.45 (s, 3 H, Me), 6.90 (s, 2H, Arom-H), 6.95 (d, $^3J_{HH}$=7.2 Hz, 4H, Arom-H), 7.95 (t, $^3J_{HH}$=8.0 Hz, 1H, Py-H), 8.23 (d, $^3J_{HH}$=7.2 Hz, 4H, Arom-H), 8.30 (d, $^3J_{HH}$=8.0 Hz, 1H, Py-H), 8.50 (d, $^3J_{HH}$=8.0 Hz, 1H, Py-

H). $^{13}C$ NMR (500 MHz, $CD_2Cl_2$, TMS (selected signals)): δ 167.5 (C=N), 169.0 (C=N). Anal. Calculated for $C_{24}H_{24}N_4O_2$ (Mol. Wt.: 400.47): C, 71.98; H, 6.04; N, 13.99. Found: C, 72.10; H, 6.27; N, 14.16.

Example 4

2,6-Bis(1-(2-methyl-3-nitrophenylimino)ethyl)pyridine (**9**)

1-(6-Acetyl-pyridin-2-yl)-ethanone (12.07 g, 0.074 mol), 25.0 g (0.164 mol) of 2-methyl-3-nitro-phenylamine, 300 ml of the toluene and a few crystals of *p*-toluenesulfonic acid were refluxed under a flow of nitrogen using a Dean-Stark trap for 3 days until the calculated amount of the water was separated (2.66 ml). The solvent was removed in a rotary evaporator and the resultant reaction mixture was recrystallized from 50 ml of ethanol. The yield of **9** was 24.60 g (77%) as a pale yellow solid. Compound **9** is stable at the room temperature. $^1H$ NMR (500 MHz, $CD_2Cl_2$, TMS): δ 2.10 (s, 6 H, Me), 2.25 (s, 6 H, Me), 6.60 (d, $^3J_{HH}$=7.0 Hz, 2H, Arom-H), 7.15 (t, $^3J_{HH}$=7.0 Hz, 2H, Arom-H), 6.43 (d, $^3J_{HH}$=7.0 Hz, 2H, Arom-H), 7.48 (m, 4H, Arom-H), 7.80 (t, $^3J_{HH}$=8.0 Hz, 1H, Py-H), 8.30 (d, $^3J_{HH}$=8.0 Hz, 1H, Py-H). $^{13}C$ NMR (500 MHz, $CD_2Cl_2$ (selected signals)): δ 169.0 (C=N). Anal. Calculated for $C_{23}H_{21}N_5O_4$ (Mol. Wt.: 431.44): C, 64.03; H, 4.91; N. 16.23. Found: C, 64.11; H, 4.95; N. 16.32.

Example 5

2,6-Bis(1-(2-methyl-4-nitrophenylimino)ethyl)pyridine iron (II) chloride (**10**)

Anhydrous iron(II) chloride (1.0 g, 0.00789 mol) was dissolved in 60 ml of warm n-butanol. Then, 3.57 g (0.00828 mol) of **6** were added in one portion to the reaction mixture. The mixture was kept at 40° C for and additional h, and then was cooled to ambient temperature. The resultant blue precipitate was filtered and washed twice by 20 ml of pentane and dried under vacuum. Yield of **10** was 3.83 g (87%). Anal. Calculated for $C_{23}H_{21}Cl_2FeN_5O_4$ (Mol. Wt.: 558.19): C, 49.49; H, 3.79; N, 12.55. Found: C, 49.63; H, 3.82; N, 12.70. Direct probe MS: Exact Mass for $C_{23}H_{21}Cl_2FeN_5O_4$: 557.03. Found 557.03.

Example 6

2,6-Bis(1-(2-methyl-3-nitrophenylimino)ethyl)pyridine iron (II) chloride (**12**)

Anhydrous iron(II) chloride (1.0 g, 0.00789 mol) was dissolved in 60 ml of warm *n*-butanol. Then, 3.57 g (0.00828 mol) of **6** were added in one portion to the reaction mixture. The mixture was kept at 40° C for an hour and then was cooled to ambient temperature. The resultant blue precipitate was filtered and washed twice with 20 ml of pentane and dried under vacuum. Yield of **12** was 4.10 g (93%). Anal. Calculated for $C_{23}H_{21}Cl_2FeN_5O_4$ (Mol. Wt.: 558.19): C, 49.49; H, 3.79; N, 12.55. Found: C, 49.51; H, 3.91; N, 12.68. Direct probe MS: Exact Mass for $C_{23}H_{21}Cl_2FeN_5O_4$: 557.03. Found 557.03.

Example 7

(1-{6-[1-(4-Nitro-phenylimino)-ethyl]-pyridin-2-yl}-ethylidene)-(2,4,6-trimethyl-phenyl)-amine iron(II) chloride (**14**)

(1-{6-[1-(4-Nitro-phenylimino)-ethyl]-pyridin-2-yl}-ethylidene)-(2,4,6-trimethyl-phenyl)-amine (**8**) (1.36 g, 0.00339 mol) were dissolved in 40 ml of THF. Iron(II) chloride tetrahydrate (0.60 g, 0.003 mol) was added to the reaction mixture in one portion. The resultant blue precipitate was filtered after 12 h of stirring, washed twice with 20 ml of pentane, and dried under vacuum. Yield of **14** was 1.34 g (84%). Anal. Calculated for $C_{24}H_{24}Cl_2FeN_4O_2$ (Mol. Wt.: 527.22): C, 54.67; H, 4.59; N, 10.63. Found: C, 54.80; H, 4.73; N, 10.64. Direct probe MS: Exact Mass for $C_{24}H_{24}Cl_2FeN_4O_2$: Exact Mass: 526.06. Found Exact Mass: 526.06.

Examples 8-12 and Comparative Example A

**General conditions of the oligomerizations.** Ethylene oligomerizations are done in a 1-liter stainless steel Zipperclave® (Autoclave Engineers, Erie, PA 16509, USA). The iron complex and cocatalyst are charged separately using stainless steel injection tubes. The steps for a typical oligomerization follow. The injectors are charged in a glovebox. The cocatalyst, MMAO (modified methylalumoxane, having some n-butyl groups in place of methyl groups, obtained from Akzo-Nobel Inc., Chicago, IL 60606 USA) was obtained as a 7-wt% solution in o-xylene and was charged as such into the injector assembly along with 10 ml chase of o-xylene. The iron complexes are prepared as suspensions in o-xylene (10mg/100 ml). A sample was pulled from a well-stirred suspension of the iron complex and was added to a 10 ml charge of o-xylene. The injectors were attached to autoclave ports equipped with dip tubes. Nitrogen was sparged through the loose fittings at the attachment points prior to making them tight. The desired charge of o-xylene was then pressured into the autoclave. The agitator and heater were turned on. When the desired temperature was reached, the cocatalyst was charged to the clave by blowing ethylene down through the cocatalyst injector. After a significant pressure rise was seen in the autoclave to indicate the cocatalyst and chase solvent had entered, the injector was isolated from the process using its valves. The pressure controller was then set to 34.5 kPa below the desired ethylene operating pressure and was put into the automatic mode to allow it to control the operation of the ethylene addition valve. When the pressure was 34.5 kPa below the desired operating pressure, the controller is put into manual mode and the valve was set to 0% output. When the batch temperature was stable and at the desired value, the suspension of the iron complex was injected using enough nitrogen such that the pressure was boosted to the desired operating pressure. At the same time as the iron complex injection, the pressure controller was put in the automatic mode and the oligomerization was underway. The 34.5 kPa boost was obtained routinely by having a small reservoir between the nitrogen source and the catalyst injector. A valve was closed between the nitrogen source and the reservoir prior to injecting the iron complex so the same volume of nitrogen was used each time to inject the iron complex suspension. During the oligomerization, the ethylene flow was recorded. The flow meter was calibrated in L/min at room temperature and pressure, and is useful especially comparative results. To stop the oligomerization, the pressure controller was put into manual, the ethylene valve was closed, and the reactor was cooled.

All oligomerizations were carried out at 120°C, 4.83 MPa (gauge) ethylene pressure, in a mixed xylenes solvent. Schulz-Flory constants ("K") for the complexes were determined based on the slopes of the Schulz-Flory plots of weight fraction data determined by GC for $C_6$ to $C_{28}$. The "% Solids" is [(weight of xylenes insoluble fraction at about 23°C)/(weight insolubles)+(weight of solubles)]x100. The weight of solubles is determined by gas chromatography. Table 1 gives the results of the oligomerizations. For the reader's convenience, the structures of the various iron complexes in Table 1 are shown below.

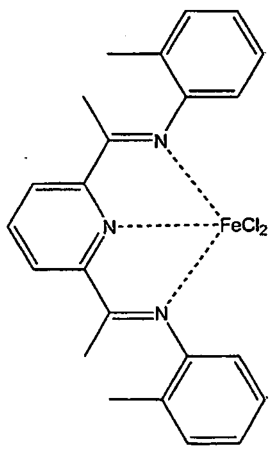

1

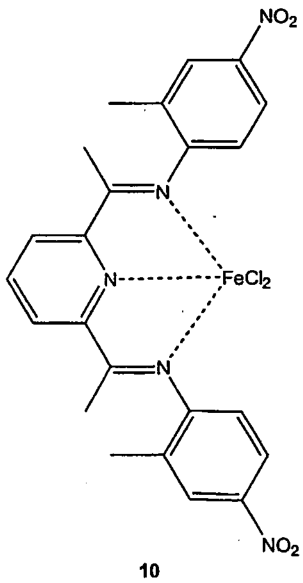

10

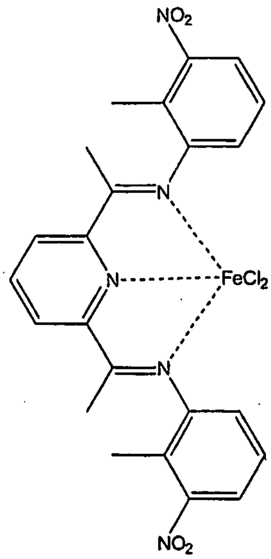

12

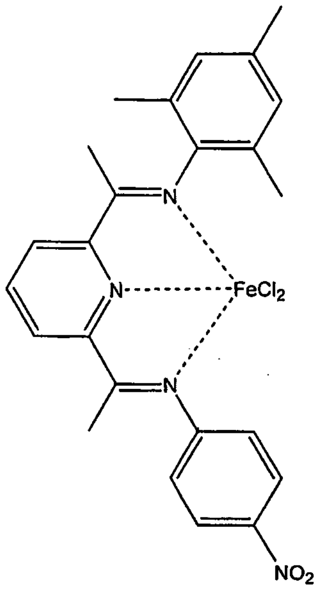

14

Table 1

| Ex | Iron complex, μmoles | Amount of MMAO, mmoles | "K" | Kg of LAO per g of catalyst | % Solids |
|---|---|---|---|---|---|
| | | | | | |
| 8 | **10,** 0.35 | 0.70 | 0.65 | 86 | 10.43 |
| 9 | **10,** 0.18 | 0.70 | 0.64 | 81 | 10.58 |
| 10 | **12,** 0.086 | 0.35 | 0.62 | 134 | 8.62 |
| 11 | **14,** 0.19 | 0.70 | 0.64 | 113 | 2.93 |
| 12 | **14,** 0.15 | 0.70 | 0.62 | 68 | 6.35 |
| A | **1,** 0.071 | 0.70 | 0.58 | 322 | 5.59 |

Table 2 gives the ethylene flow in L/min with time (minutes) for each oligomerization example. This data shows that complexes containing nitro groups are longer lasting than a complex that does not contain a nitro group.

Table 2

| Example | 8 | 9 | 10 | 11 | 12 | A |
|---|---|---|---|---|---|---|
| | | | | | | |
| Time | E Flow | E Flow | E Flow | E Flow | E Flow | E Flow |
| 0.05 | 0.17 | 0.27 | 0.31 | 3.82 | 0.07 | 1.21 |
| 0.5 | 6 | 8.6 | 1.12 | 11.1 | 8.67 | 12.5 |
| 1 | 9.08 | 4.85 | 3.7 | 12 | 9.22 | 10.9 |
| 1.5 | 9.4 | 3.06 | 2.03 | 7.3 | 3.52 | 7.09 |
| 2 | 8.98 | 0.39 | 1.42 | 1.89 | 0.32 | 2.87 |
| 3 | 4.75 | 0.36 | 1.21 | 0.01 | 0.06 | 0.1 |
| 6 | 0.33 | 0.36 | 0.36 | 0.03 | 0.07 | 0 |

## Claims

1. A process for the oligomerization of ethylene to linear α-olefins, comprising, contacting, at a temperature of -20°C to 200°C, ethylene and an Fe, Co, Cr or V complex of a ligand of the formula

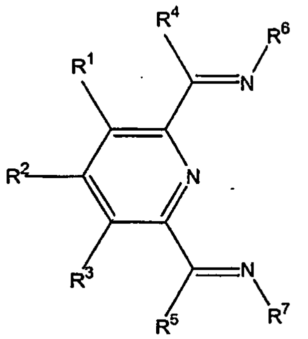

(I)

wherein:

$R^1$, $R^2$ and $R^3$ are each independently hydrogen, hydrocarbyl, substituted hydrocarbyl or an inert functional group, provided that any two of $R^1$, $R^2$ and $R^3$ vicinal to one another taken together may form a ring;
$R^4$ and $R^5$ are each independently hydrogen, hydrocarbyl, substituted hydrocarbyl or an inert functional group provided that $R^1$ and $R^4$ and/or $R^3$ and $R^5$ taken together may form a ring;
$R^6$ and $R^7$ are each independently aryl or substituted aryl having a first ring atom bound to the imino nitrogen, provided that at least one of $R^6$ and $R^7$ is substituted with at least one nitro group.

2. The process as recited in claim 1 wherein said complex is an Fe complex.

3. The process as recited in claim 1 or 2 wherein:

in $R^6$, a second ring atom adjacent to said first ring atom is bound to a halogen, a primary carbon group, a secondary carbon group or a tertiary carbon group; and further provided that
in $R^6$, when said second ring atom is bound to a halogen or a primary carbon group, none, one or two of the other ring atoms in $R^6$ and $R^7$ adjacent to said first ring atom are bound to a halogen or a primary carbon group, with the remainder of the ring atoms adjacent to said first ring atom being bound to a hydrogen atom; or
in $R^6$, when said second ring atom is bound to a secondary carbon group, none, one or two of the other ring atoms in $R^6$ and $R^7$ adjacent to said first ring atom are bound to a halogen, a primary carbon group or a secondary carbon group, with the remainder of the ring atoms adjacent to said first ring atom being bound to a hydrogen atom; or
in $R^6$, when said second ring atom is bound to a tertiary carbon group, none or one of the other ring atoms in $R^6$ and $R^7$ adjacent to said first ring atom are bound to a tertiary carbon group, with the remainder of the ring atoms adjacent to said first ring atom being bound to a hydrogen atom.

4. The process as recited in claim 1, 2 or 3 wherein $R^6$ is

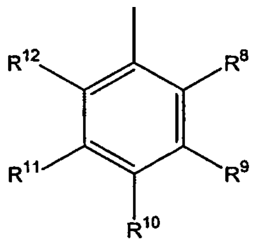

(II)

and $R^7$ is

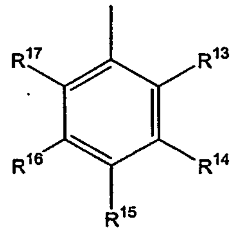

(III).

wherein $R^8$, $R^9$, $R^{10}$, $R^{11}$, $R^{12}$, $R^{13}$, $R^{14}$, $R^{15}$, $R^{16}$, and $R^{17}$ are hydrocarbyl, substituted hydrocarbyl or a functional group.

5. The process as recited in claim 4 wherein at least one nitro group is present in each of (II) and (III), and wherein at least one of $R^9$, $R^{10}$, and $R^{11}$ is nitro, and at least one of $R^{14}$, $R^{15}$ and $R^{16}$ is nitro.

6. A compound of the formula

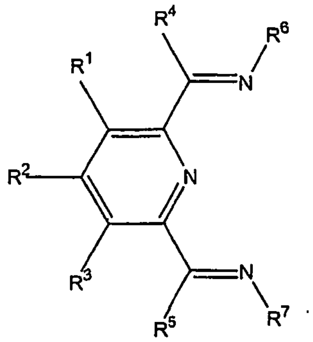

wherein:

$R^1$, $R^2$ and $R^3$ are each independently hydrogen, hydrocarbyl, substituted hydrocarbyl or an inert functional group, provided that any two of $R^1$, $R^2$ and $R^3$ vicinal to one another taken together may form a ring;
$R^4$ and $R^5$ are each independently hydrogen, hydrocarbyl, substituted hydrocarbyl or an inert functional group provided that $R^1$ and $R^4$ and/or $R^3$ and $R^5$ taken together may form a ring;
$R^6$ and $R^7$ are each independently aryl or substituted aryl having a first ring atom bound to the imino nitrogen, provided that at least one of $R^6$ and $R^7$ is substituted with at least one nitro group, and provided that:

in $R^6$, a second ring atom adjacent to said first ring atom is bound to a halogen, a primary carbon group, a secondary carbon group or a tertiary carbon group; and further provided that
in $R^6$, when said second ring atom is bound to a halogen or a primary carbon group, none, one or two of the other ring atoms in $R^6$ and $R^7$ adjacent to said first ring atom are bound to a halogen or a primary carbon group, with the remainder of the ring atoms adjacent to said first ring atom being bound to a hydrogen atom; or
in $R^6$, when said second ring atom is bound to a secondary carbon group, none, one or two of the other ring atoms in $R^6$ and $R^7$ adjacent to said first ring atom are bound to a halogen, a primary carbon group or a secondary carbon group, with the remainder of the ring atoms adjacent to said first ring atom being bound to a hydrogen atom; or
in $R^6$, when said second ring atom is bound to a tertiary carbon group, none or one of the other ring atoms in $R^6$ and $R^7$ adjacent to said first ring atom are bound to a tertiary carbon group, with the remainder of the ring atoms adjacent to said first ring atom being bound to a hydrogen atom.

7. The compound as recited in claim 6 wherein $R^6$ is

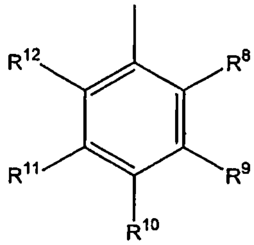

(II)

and $R^7$ is

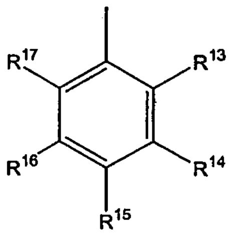

(III).

wherein $R^8$, $R^9$, $R^{10}$, $R^{11}$, $R^{12}$, $R^{13}$, $R^{14}$, $R^{15}$, $R^{16}$ , and $R^{17}$ are hydrocarbyl, substituted hydrocarbyl or a functional group.

**8.** The compound as recited in claim 7 wherein at least one nitro group is present in each of (II and (III), at least one of $R^9$, $R^{10}$, and $R^{11}$ is nitro, and at least one of $R^{14}$ , $R^{15}$ and $R^{16}$ is nitro.

**9.** A compound of the formula

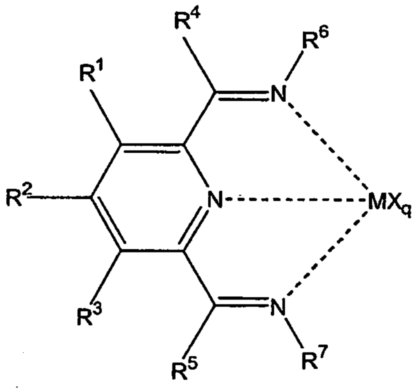

wherein:

each X is independently a monoanion;
M is a transition metal selected from the group consisting of Fe, Co, Cr and V;
q is an oxidation state of said transition metal;
$R^1$, $R^2$ and $R^3$ are each independently hydrogen, hydrocarbyl, substituted hydrocarbyl or an inert functional group, provided that any two of $R^1$, $R^2$ and $R^3$ vicinal to one another taken together may form a ring;
$R^4$ and $R^5$ are each independently hydrogen, hydrocarbyl, substituted hydrocarbyl or an inert functional group provided that $R^1$ and $R^4$ and/or $R^3$ and $R^5$ taken together may form a ring;
$R^6$ and $R^7$ are each independently aryl or substituted aryl having a first ring atom bound to the imino nitrogen, provided that at least one of $R^6$ and $R^7$ is substituted with at least one nitro group, and provided that: in $R^6$, a second ring atom adjacent to said first ring atom is bound to a halogen, a primary carbon group, a secondary carbon group or a tertiary carbon group; and further provided that

in $R^6$, when said second ring atom is bound to a halogen or a primary carbon group, none, one or two of the other ring atoms in $R^6$ and $R^7$ adjacent to said first ring atom are bound to a halogen or a primary carbon group, with the remainder of the ring atoms adjacent to said first ring atom being bound to a hydrogen atom; or
in $R^6$, when said second ring atom is bound to a secondary carbon group, none, one or two of the other ring atoms in $R^6$ and $R^7$ adjacent to said first ring atom are bound to a halogen, a primary carbon group or a secondary carbon group, with the remainder of the ring atoms adjacent to said first ring atom being bound to a hydrogen atom; or
in $R^6$, when said second ring atom is bound to a tertiary carbon group, none or one of the other ring atoms in $R^6$ and $R^7$ adjacent to said first ring atom are bound to a tertiary carbon group, with the remainder of the ring atoms adjacent to said first ring atom being bound to a hydrogen atom.

**10.** The compound as recited in claim 9 wherein M is Fe.

**11.** The compound as recited in claim 9 or 10 wherein $R^6$ is

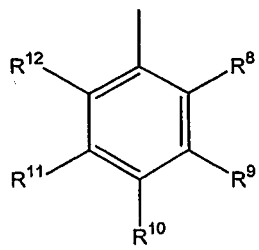

(II)

and $R^7$ is

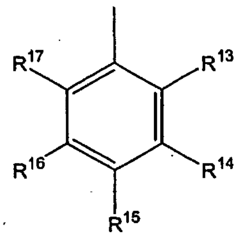

(III).

wherein $R^8$, $R^9$, $R^{10}$, $R^{11}$, $R^{12}$, $R^{13}$, $R^{14}$, $R^{15}$, $R^{16}$, and $R^{17}$ are hydrocarbyl, substituted hydrocarbyl or a functional group.

**12.** The compound as recited in claim 11 wherein at least one nitro group is present in each of (II) and (III), at least one of $R^9$, $R^{10}$, and $R^{11}$ is nitro, and at least one of $R^{14}$, $R^{15}$ and $R^{16}$ is nitro.

## Patentansprüche

**1.** Verfahren zur Oligomerisierung von Ethylen zu linearen α-Olefinen, umfassend das in Kontakt bringen, bei einer Temperatur von -20°C bis 200°C, von Ethylen und einem Fe-, Co-, Cr- oder V-Komplex eines Liganden der folgenden Formel:

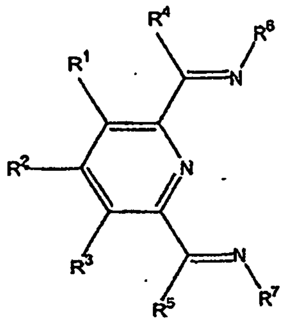

(I)

worin:

$R^1$, $R^2$ und $R^3$ jeweils unabhängig Wasserstoff, Hydrocarbyl, substituiertes Hydrocarbyl oder eine inerte funktionelle Gruppe darstellen, vorausgesetzt, dass jedwede zwei von $R^1$, $R^2$ und $R^3$ vizinal zueinander zusammengenommen einen Ring bilden können;
$R^4$ und $R^5$ jeweils unabhängig Wasserstoff, Hydrocarbyl, substituiertes Hydrocarbyl oder eine inerte funktionelle Gruppe darstellen, vorausgesetzt, dass $R^1$ und $R^4$ und/oder $R^3$ und $R^5$ zusammengenommen einen Ring bilden können;
$R^6$ und $R^7$ jeweils unabhängig Aryl oder substituiertes Aryl mit einem an den Iminostickstoff gebundenen ersten Ringatom darstellen, vorausgesetzt, dass mindestens eines von $R^6$ und $R^7$ durch mindestens eine Nitrogruppe substituiert ist.

**2.** Verfahren nach Anspruch 1, worin der Komplex einen Fe-Komplex darstellt.

**3.** Verfahren nach Anspruch 1 oder 2, worin:

in $R^6$, ein zweites Ringatom benachbart zum ersten Ringatom an ein Halogen, eine primäre Kohlenstoffgruppe, eine sekundäre Kohlenstoffgruppe oder eine tertiäre Kohlenstoffgruppe gebunden ist; und weiter vorausgesetzt, dass
in $R^6$, wenn das zweite Ringatom an ein Halogen oder eine primäre Kohlenstoffgruppe gebunden ist, keines, eines oder zwei der anderen Ringatome in $R^6$ und $R^7$ benachbart zum ersten Ringatom an ein Halogen oder eine primäre Kohlenstoffgruppe gebunden ist/sind, wobei die übrigen der Ringatome benachbart zum ersten Ringatom an ein Wasserstoffatom gebunden sind; oder
in $R^6$, wenn das zweite Ringatom an eine sekundäre Kohlenstoffgruppe gebunden ist, keines, eines oder zwei der anderen Ringatome in $R^6$ und $R^7$ benachbart zum ersten Ringatom an ein Halogen, eine primäre Kohlenstoffgruppe oder eine sekundäre Kohlenstoffgruppe gebunden ist/sind, wobei die übrigen der Ringatome benachbart zum ersten Ringatom an ein Wasserstoffatom gebunden sind; oder
in $R^6$, wenn das zweite Ringatom an eine tertiäre Kohlenstoffgruppe gebunden ist, keines oder eines der anderen Ringatome in $R^6$ und $R^7$ benachbart zum ersten Ringatom an eine tertiäre Kohlenstoffgruppe gebunden ist, wobei die übrigen der Ringatome benachbart zum ersten Ringatom an ein Wasserstoffatom gebunden sind.

**4.** Verfahren nach Anspruch 1, 2 oder 3, worin $R^6$ Folgendes darstellt:

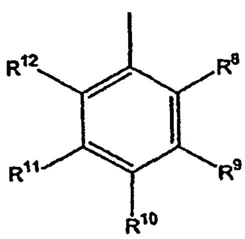

(II) )

und $R^7$ Folgendes darstellt:

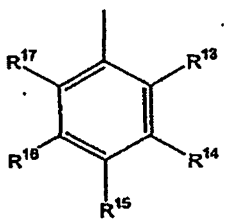

(III).

worin $R^8$, $R^9$, $R^{10}$, $R^{11}$, $R^{12}$, $R^{13}$, $R^{14}$, $R^{15}$, $R^{16}$ und $R^{17}$ Hydrocarbyl, substituiertes Hydrocarbyl oder eine funktionelle Gruppe darstellen.

**5.** Verfahren nach Anspruch 4, worin mindestens eine Nitrogruppe in jedem von (II) und (III) anwesend ist, und worin mindestens eines von $R^9$, $R^{10}$ und $R^{11}$ Nitro darstellt und mindestens eines von $R^{14}$, $R^{15}$ und $R^{16}$ Nitro darstellt.

**6.** Verbindung der folgenden Formel:

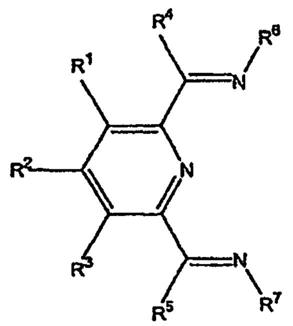

worin:

$R^1$, $R^2$ und $R^3$ jeweils unabhängig Wasserstoff, Hydrocarbyl, substituiertes Hydrocarbyl oder eine inerte funktionelle Gruppe darstellen, vorausgesetzt, dass jedwede zwei von $R^1$, $R^2$ und $R^3$ vizinal zueinander zusammengenommen einen Ring bilden können;
$R^4$ und $R^5$ jeweils unabhängig Wasserstoff, Hydrocarbyl, substituiertes Hydrocarbyl oder eine inerte funktionelle Gruppe darstellen, vorausgesetzt, dass $R^1$ und $R^4$ und/oder $R^3$ und $R^5$ zusammengenommen einen Ring bilden können;
$R^6$ und $R^7$ jeweils unabhängig Aryl oder substituiertes Aryl mit einem an den Iminostickstoff gebundenen ersten Ringatom darstellen, vorausgesetzt, dass mindestens eines von $R^6$ und $R^7$ durch mindestens eine Nitrogruppe substituiert ist, und vorausgesetzt, dass:

in $R^6$ ein zweites Ringatom benachbart zum ersten Ringatom an ein Halogen, eine primäre Kohlenstoffgruppe, eine sekundäre Kohlenstoffgruppe oder eine tertiäre Kohlenstoffgruppe gebunden ist; und weiter vorausgesetzt, dass
in $R^6$, wenn das zweite Ringatom an ein Halogen oder eine primäre Kohlenstoffgruppe gebunden ist, keines, eines oder zwei der anderen Ringatome in $R^6$ und $R^7$ benachbart zum ersten Ringatom an ein Halogen oder eine primäre Kohlenstoffgruppe gebunden ist/sind, wobei die übrigen der Ringatome benachbart zum ersten Ringatom an ein Wasserstoffatom gebunden sind; oder
in $R^6$, wenn das zweite Ringatom an eine sekundäre Kohlenstoffgruppe gebunden ist, keines, eines oder zwei der anderen Ringatome in $R^6$ und $R^7$ benachbart zum ersten Ringatom an ein Halogen, eine primäre Kohlenstoffgruppe oder eine sekundäre Kohlenstoffgruppe gebunden ist/sind, wobei die übrigen der Ringatome benachbart zum ersten Ringatom an ein Wasserstoffatom gebunden sind; oder
in $R^6$, wenn das zweite Ringatom an eine tertiäre Kohlenstoffgruppe gebunden ist, keines oder eines der anderen Ringatome in $R^6$ und $R^7$ benachbart zum ersten Ringatom an eine tertiäre Kohlenstoffgruppe gebunden ist, wobei die übrigen der Ringatome benachbart zum ersten Ringatom an ein Wasserstoffatom gebunden sind.

7. Verbindung nach Anspruch 6, worin $R^6$ Folgendes darstellt:

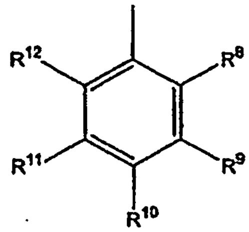

(II)

und $R^7$ Folgendes darstellt:

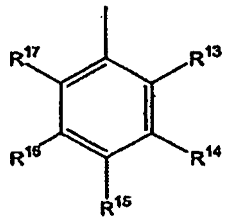

(III).

worin $R^8$, $R^9$, $R^{10}$, $R^{11}$, $R^{12}$, $R^{13}$, $R^{14}$, $R^{15}$, $R^{16}$ und $R^{17}$ Hydrocarbyl, substituiertes Hydrocarbyl oder eine funktionelle Gruppe darstellen.

8. Verbindung nach Anspruch 7, worin in jedem von (II) und (III) mindestens eine Nitrogruppe anwesend ist, mindestens eines von $R^9$, $R^{10}$ und $R^{11}$ Nitro darstellt und mindestens eines von $R^{14}$, $R^{15}$ und $R^{16}$ Nitro darstellt.

9. Verbindung der folgenden Formel:

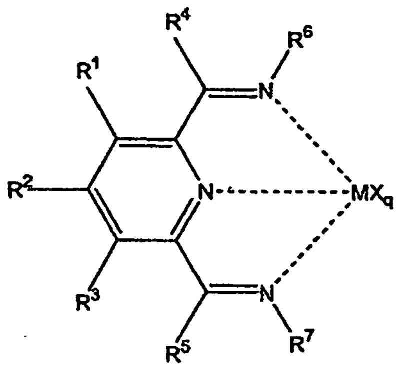

worin:

jedes X unabhängig ein Monoanion darstellt;
M ein Übergangsmetall darstellt, das aus der Gruppe ausgewählt ist, bestehend aus Fe, Co, Cr und V;
q einen Oxidationszustand des Übergangsmetalls darstellt;
$R^1$, $R^2$ und $R^3$ jeweils unabhängig Wasserstoff, Hydrocarbyl, substituiertes Hydrocarbyl oder eine inerte funktionelle Gruppe darstellen, vorausgesetzt, dass jedwede zwei von $R^1$, $R^2$ und $R^3$ vizinal zueinander zusammengenommen einen Ring bilden können;
$R^4$ und $R^5$ jeweils unabhängig Wasserstoff, Hydrocarbyl, substituiertes Hydrocarbyl oder eine inerte funktionelle Gruppe darstellen, vorausgesetzt, dass $R^1$ und $R^4$ und/oder $R^3$ und $R^5$ zusammengenommen einen Ring bilden können;
$R^6$ und $R^7$ jeweils unabhängig Aryl oder substituiertes Aryl mit einem an den Iminostickstoff gebundenen ersten

Ringatom darstellen, vorausgesetzt, dass mindestens eines von $R^6$ und $R^7$ durch mindestens eine Nitrogruppe substituiert ist, und vorausgesetzt, dass: in $R^6$ ein zweites Ringatom benachbart zum ersten Ringatom an ein Halogen, eine primäre Kohlenstoffgruppe, eine sekundäre Kohlenstoffgruppe oder eine tertiäre Kohlenstoffgruppe gebunden ist, und weiter vorausgesetzt, dass

in $R^6$, wenn das zweite Ringatom an ein Halogen oder eine primäre Kohlenstoffgruppe gebunden ist, keines, eines oder zwei der anderen Ringatome in $R^6$ und $R^7$ benachbart zum ersten Ringatom an ein Halogen oder eine primäre Kohlenstoffgruppe gebunden ist/sind, wobei die übrigen der Ringatome benachbart zum ersten Ringatom an ein Wasserstoffatom gebunden sind; oder
in $R^6$, wenn das zweite Ringatom an eine sekundäre Kohlenstoffgruppe gebunden ist, keines, eines oder zwei der anderen Ringatome in $R^6$ und $R^7$ benachbart zum ersten Ringatom an ein Halogen, ein primäre Kohlenstoffgruppe oder eine sekundäre Kohlenstoffgruppe gebunden ist/sind, wobei die übrigen der Ringatome benachbart zum ersten Ringatom an ein Wasserstoffatom gebunden sind; oder
in $R^6$, wenn das zweite Ringatom an eine tertiäre Kohlenstoffgruppe gebunden ist, keines oder eines der anderen Ringatome in $R^6$ und $R^7$ benachbart zum ersten Ringatom an eine tertiäre Kohlenstoffgruppe gebunden ist, wobei die übrigen der Ringatome benachbart zum ersten Ringatom an ein Wasserstoffatom gebunden sind.

**10.** Verbindung nach Anspruch 9, worin M für Fe steht.

**11.** Verbindung nach Anspruch 9 oder 10, worin $R^6$ Folgendes darstellt:

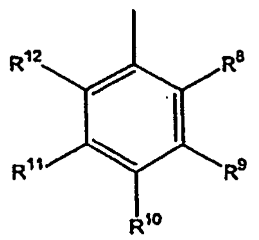

**(II)**

und $R^7$ Folgendes darstellt:

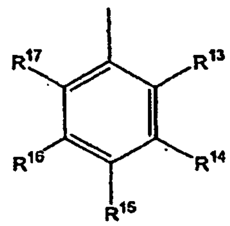

**(III).**

worin $R^8$, $R^9$, $R^{10}$, $R^{11}$, $R^{12}$, $R^{13}$, $R^{14}$, $R^{15}$, $R^{16}$ und $R^{17}$ Hydrocarbyl, substituiertes Hydrocarbyl oder eine funktionelle Gruppe darstellen.

**12.** Verbindung nach Anspruch 11, worin in jedem von (II) und (III) mindestens eine Nitrogruppe anwesend ist, mindestens eines von $R^9$, $R^{10}$ und $R^{11}$ Nitro darstellt und mindestens eines von $R^{14}$, $R^{15}$ und $R^{16}$ Nitro darstellt.

**Revendications**

**1.** Procédé pour l'oligomérisation d'éthylène en $\alpha$-oléfines linéaires, comprenant la mise en contact, à une température de -20°C à 200°C, d'éthylène et d'un complexe de Fe, Co, Cr ou V d'un ligand de la formule:

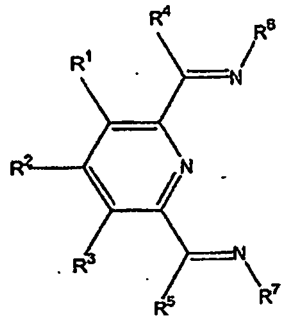

(I)

dans laquelle:

$R^1$, $R^2$ et $R^3$ sont chacun indépendamment un hydrogène, un groupe hydrocarbyle, un groupe hydrocarbyle substitué ou un groupe fonctionnel inerte, à condition que deux quelconques parmi $R^1$, $R^2$ et $R^3$ voisins l'un avec l'autre pris ensemble puissent former un cycle;
$R^4$ et $R^5$ sont chacun indépendamment un hydrogène, un groupe hydrocarbyle, un groupe hydrocarbyle substitué ou un groupe fonctionnel inerte, à condition que $R^1$ et $R^4$ et/ou $R^3$ et $R^5$ pris ensemble puissent former un cycle;
$R^6$ et $R^7$ sont chacun indépendamment un groupe aryle ou un groupe aryle substitué possédant un premier atome de cycle lié à l'azote du groupe imino, à condition que au moins un parmi $R^6$ et $R^7$ soit substitué avec au moins un groupe nitro.

**2.** Procédé selon la revendication 1, dans lequel ledit complexe est un complexe de Fe.

**3.** Procédé selon la revendication 1 ou 2, dans lequel:

dans $R^6$, un deuxième atome de cycle adjacent audit premier atome de cycle est lié à un halogène, un groupe de carbone primaire, un groupe de carbone secondaire ou un groupe de carbone tertiaire; et en outre à condition que:
dans $R^6$, lorsque ledit deuxième atome de cycle est lié à un halogène ou un groupe de carbone primaire, aucun, un ou deux des autres atomes de cycle dans $R^6$ et $R^7$ adjacents audit premier atome de cycle soient liés à un halogène ou un groupe de carbone primaire, le reste des atomes de cycle adjacents audit premier atome de cycle étant lié à un atome d'hydrogène; ou
dans $R^6$, lorsque ledit deuxième atome de cycle est lié à un groupe de carbone secondaire, aucun, un ou deux des autres atomes de cycle dans $R^6$ et $R^7$ adjacents audit premier atome de cycle soient liés à un halogène, un groupe de carbone primaire ou un groupe de carbone secondaire, le reste des atomes de cycle adjacents audit premier atome de cycle étant lié à un atome d'hydrogène; ou
dans $R^6$, lorsque ledit deuxième atome de cycle est lié à un groupe de carbone tertiaire, aucun ou un des autres atomes de cycle dans $R^6$ et $R^7$ adjacent audit premier atome de cycle soit lié à un groupe de carbone tertiaire, le reste des atomes de cycle adjacents audit premier atome de cycle étant lié à un atome d'hydrogène.

**4.** Procédé selon la revendication 1, 2 ou 3, dans lequel $R^6$ est:

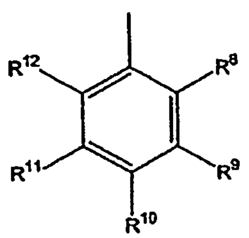

(II)

et $R^7$ est:

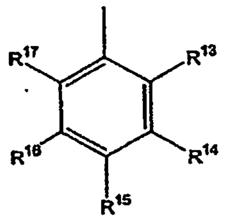

(III).

dans lesquelles formules, $R^8$, $R^9$, $R^{10}$, $R^{11}$, $R^{12}$, $R^{13}$, $R^{14}$, $R^{15}$, $R^{16}$ et $R^{17}$ sont un groupe hydrocarbyle, un groupe hydrocarbyle substitué ou un groupe fonctionnel.

**5.** Procédé selon la revendication 4, dans lequel au moins un groupe nitro est présent dans chacune des formules (II) et (III) et dans lequel au moins un parmi $R^9$, $R^{10}$ et $R^{11}$ est un groupe nitro et au moins un parmi $R^{14}$, $R^{15}$ et $R^{16}$ est un groupe nitro.

**6.** Composé de la formule:

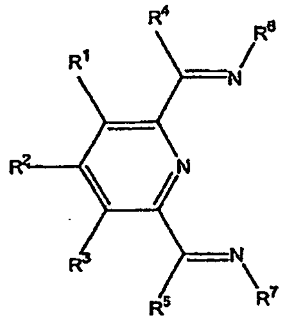

dans laquelle:

$R^1$, $R^2$ et $R^3$ sont chacun indépendamment un hydrogène, un groupe hydrocarbyle, un groupe hydrocarbyle substitué ou un groupe fonctionnel inerte, à condition que deux quelconques parmi $R^1$, $R^2$ et $R^3$ voisins l'un avec l'autre pris ensemble puissent former un cycle;
$R^4$ et $R^5$ sont chacun indépendamment un hydrogène, un groupe hydrocarbyle, un groupe hydrocarbyle substitué ou un groupe fonctionnel inerte, à condition que $R^1$ et $R^4$ et/ou $R^3$ et $R^5$ pris ensemble puissent former un cycle;
$R^6$ et $R^7$ sont chacun indépendamment un groupe aryle ou un groupe aryle substitué possédant un premier atome de cycle lié à l'azote du groupe imino, à condition que au moins un parmi $R^6$ et $R^7$ soit substitué avec au moins un groupe nitro, et à condition que:

dans $R^6$, un deuxième atome de cycle adjacent audit premier atome de cycle soit lié à un halogène, un groupe de carbone primaire, un groupe de carbone secondaire ou un groupe de carbone tertiaire; et en outre à condition que:
dans $R^6$, lorsque ledit deuxième atome de cycle est lié à un halogène ou un groupe de carbone primaire, aucun, un ou deux des autres atomes de cycle dans $R^6$ et $R^7$ adjacents audit premier atome de cycle soient liés à un halogène ou un groupe de carbone primaire, le reste des atomes de cycle adjacents audit premier atome de cycle étant lié à un atome d'hydrogène; ou
dans $R^6$, lorsque ledit deuxième atome de cycle est lié à un groupe de carbone secondaire, aucun, un ou deux des autres atomes de cycle dans $R^6$ et $R^7$ adjacents audit premier atome de cycle soient liés à un halogène, un groupe de carbone primaire ou un groupe de carbone secondaire, le reste des atomes de cycle adjacents audit premier atome de cycle étant lié à un atome d'hydrogène; ou
dans $R^6$, lorsque ledit deuxième atome de cycle est lié à un groupe de carbone tertiaire, aucun ou un des autres atomes de cycle dans $R^6$ et $R^7$ adjacent audit premier atome de cycle soit lié à un groupe de carbone tertiaire, le reste des atomes de cycle adjacents audit premier atome de cycle étant lié à un atome d'hydrogène.

**7.** Composé selon la revendication 6, dans lequel $R^6$ est:

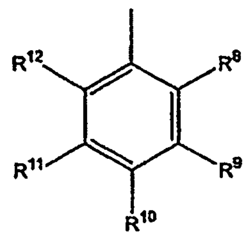

(II)

et $R^7$ est:

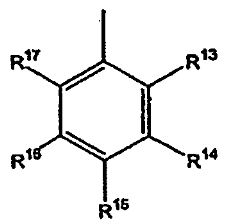

(III).

dans lesquelles formules, $R^8$, $R^9$, $R^{10}$, $R^{11}$, $R^{12}$, $R^{13}$, $R^{14}$, $R^{15}$, $R^{16}$ et $R^{17}$ sont un groupe hydrocarbyle, un groupe hydrocarbyle substitué ou un groupe fonctionnel.

**8.** Composé selon la revendication 7, dans lequel au moins un groupe nitro est présent dans chacune des formules (II) et (III), au moins un parmi $R^9$, $R^{10}$ et $R^{11}$ est un groupe nitro et au moins un parmi $R^{14}$, $R^{15}$ et $R^{16}$ est un groupe nitro.

**9.** Composé de la formule:

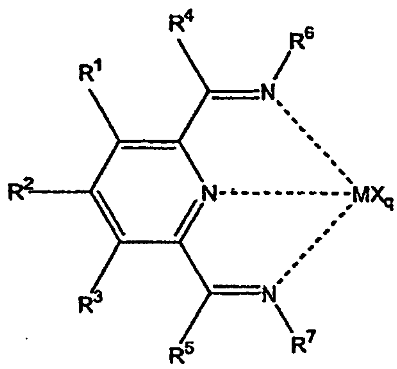

dans laquelle:

chaque X est indépendamment un monoanion;
M est un métal de transition choisi dans le groupe constitué de Fe, Co, Cr et V;
q est un état d'oxydation dudit métal de transition;
$R^1$, $R^2$ et $R^3$ sont chacun indépendamment un hydrogène, un groupe hydrocarbyle, un groupe hydrocarbyle substitué ou un groupe fonctionnel inerte, à condition que deux quelconques parmi $R^1$, $R^2$ et $R^3$ voisins l'un avec l'autre pris ensemble puissent former un cycle;
$R^4$ et $R^5$ sont chacun indépendamment un hydrogène, un groupe hydrocarbyle, un groupe hydrocarbyle substitué ou un groupe fonctionnel inerte, à condition que $R^1$ et $R^4$ et/ou $R^3$ et $R^5$ pris ensemble puissent former un cycle;
$R^6$ et $R^7$ sont chacun indépendamment un groupe aryle ou un groupe aryle substitué possédant un premier atome de cycle lié à l'azote du groupe imino, à condition que au moins un parmi $R^6$ et $R^7$ soit substitué avec

au moins un groupe nitro, et à condition que: dans $R^6$, un deuxième atome de cycle adjacent audit premier atome de cycle soit lié à un halogène, un groupe de carbone primaire, un groupe de carbone secondaire ou un groupe de carbone tertiaire; et en outre à condition que:

dans $R^6$, lorsque ledit deuxième atome de cycle est lié à un halogène ou un groupe de carbone primaire, aucun, un ou deux des autres atomes de cycle dans $R^6$ et $R^7$ adjacents audit premier atome de cycle soient liés à un halogène ou un groupe de carbone primaire, le reste des atomes de cycle adjacents audit premier atome de cycle étant lié à un atome d'hydrogène; ou

dans $R^6$, lorsque ledit deuxième atome de cycle est lié à un groupe de carbone secondaire, aucun, un ou deux des autres atomes de cycle dans $R^6$ et $R^7$ adjacents audit premier atome de cycle soient liés à un halogène, un groupe de carbone primaire ou un groupe de carbone secondaire, le reste des atomes de cycle adjacents audit premier atome de cycle étant lié à un atome d'hydrogène; ou

dans $R^6$, lorsque ledit deuxième atome de cycle est lié à un groupe de carbone tertiaire, aucun ou un des autres atomes de cycle dans $R^6$ et $R^7$ adjacent audit premier atome de cycle soit lié à un groupe de carbone tertiaire, le reste des atomes de cycle adjacents audit premier atome de cycle étant lié à un atome d'hydrogène.

**10.** Composé selon la revendication 9, dans lequel M est Fe.

**11.** Composé selon la revendication 9 ou 10, dans lequel $R^6$ est:

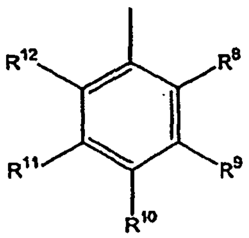

(II)

et $R^7$ est:

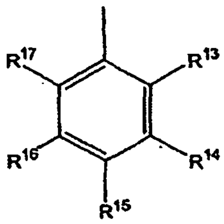

(III).

dans lesquelles formules, $R^8$, $R^9$, $R^{10}$, $R^{11}$, $R^{12}$, $R^{13}$, $R^{14}$, $R^{15}$, $R^{16}$ et $R^{17}$ sont un groupe hydrocarbyle, un groupe hydrocarbyle substitué ou un groupe fonctionnel.

**12.** Composé selon la revendication 11, dans lequel au moins un groupe nitro est présent dans chacune des formules (II) et (III), au moins un parmi $R^9$, $R^{10}$ et $R^{11}$ est un groupe nitro et au moins un parmi $R^{14}$, $R^{15}$ et $R^{16}$ est un groupe nitro.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6103946 A
- US 6534691 B
- US 6555723 B
- US 6683187 B
- US 6710006 B
- WO 04026795 A
- US 5955555 A
- US 5866663 A
- WO 9827124 A


### Non-patent literature cited in the description


- **W. BECK. et al.** *Chem. Rev.,* 1988, vol. 88, 1405-1421
- **S. H. STARES.** *Chem. Rev.,* 1993, vol. 93, 927-942
- Ullmann's Encyclopedia of Industrial Chemistry. VCH Verlagsgesellschaft mbH, 1989, vol. A13, 243-247275-276